Europäisches Patentamt

European Patent Office

Office européen des brevets

(19)

(11) Numéro de publication: **0 064 451**
**B1**

(12) **FASCICULE DE BREVET EUROPEEN**

(45) Date de publication du fascicule du brevet:
23.04.86

(51) Int. Cl.⁴: **A 61 F 13/04**

(21) Numéro de dépôt: **82400734.8**

(22) Date de dépôt: **23.04.82**

(54) **Semelle incorporable dans les bandages au plâtre du pied.**

(30) Priorité: **28.04.81 FR 8108437**

(43) Date de publication de la demande:
**10.11.82 Bulletin 82/45**

(45) Mention de la délivrance du brevet:
**23.04.86 Bulletin 86/17**

(84) Etats contractants désignés:
**AT BE CH DE GB IT LI LU NL SE**

(56) Documents cités:
**FR - A - 2 328 449**
**US - A - 3 198 192**
**US - A - 3 307 536**
**US - A - 3 680 550**
**US - A - 3 916 538**

(73) Titulaire: **Didier, Philippe, Rue Gustave Déléris,**
**Bulgneville F-88140 Contrex (FR)**
Titulaire: **Vermonet, Francis, 1 rue Kennedy,**
**F-88300 Neufchateau (FR)**

(72) Inventeur: **Didier, Philippe, Rue Gustave Déléris,**
**Bulgneville F-88140 Contrex (FR)**
Inventeur: **Vermonet, Francis, 1 rue Kennedy,**
**F-88300 Neufchateau (FR)**

(74) Mandataire: **Lemonnier, André, Cabinet**
**LEMONNIER 4, Boulevard Saint-Denis, F-75010 Paris**
**(FR)**

Il est rappelé que: Dans un délai de neuf mois à compter de la date de publication de la mention de la délivrance du brevet euro-péen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne les bandages du pied par bandes platrées dits plâtres de marche, que ceux-ci soient du type botte plâtrée montant en dessous du genou ou du type cruropédieux se prolongeant jusque sur la cuisse.

Dans le cas des plâtres de marche, il est nécessaire de mettre le plâtre à l'abri d'un contact direct avec le sol car il se produirait rapidement une fracture et une désintégration au moins partielle de la masse plâtrée. La technique connue à ce jour consiste à incorporer dans la masse du plâtre située sous le pied, des pelottes ou coussinets qui font saillie en dessous du plâtre et que l'on appelle «talonnettes de marche». Ces talonnettes de marche comportent un corps de forme générale cylindrique dont l'extrémité supérieure présente des oreilles ou bandes latérales souples qui sont enrobées à la pose dans les couches de bandes plâtrées. Avec ces talonnettes, de marche, le poids du corps est reporté sur le sol par l'intermédiaire de la section du corps cylindrique et en raison de la faible épaisseur de la masse plâtrée interposée entre le pied et la surface supérieure de la talonnette, d'une part, et de la souplesse résiduelle en résultant de la couche plâtrée interposée, d'autre part, il en résulte une compression notable de la zone de la plante du pied située au-dessus de la talonnette laquelle se traduit par une sensation d'inconfort, sinon douloureuse. En outre, lorsque le patient est dans la position assise, jambe allongée, la talonnette n'est plus au contact du sol et la partie du plâtre en arrière du talon est au contact du sol.

Dans FR-A-2 328 449 est décrit un appareil d'appui de marche constitué par une plaque qui sert de support à la plante du pied et qui comporte un quillon central, en saillie vers le bas et servant de support pendant la marche et il est prévu que cette plaque peut avoir une forme anatomique assurant un soutien optimal du pied. Cet appareil est fixé sur le pied à l'aide de bandes plâtrées mais ledit document ne propose aucune mesure pour éviter un contact direct entre les bandes plâtrées assurant la fixation et le sol.

Des moyens pour éviter ce contact direct sont suggérés par US-A-3 916 538 mais aucun moyen n'est suggéré pour éviter le contact entre les bandes plâtrées et le sol lorsque le malade est dans la position assise avec la jambe allongée.

La présente invention a pour but de munir une semelle du type décrit dans FR-A-2 328 449 d'un tel moyen.

La présente invention a pour objet une semelle pour la réalisation d'un bandage au plâtre du pied avec incorporation d'un élément d'appui pour la marche dont une partie fait saillie sous la surface inférieure du bandage, cet élément d'appui étant solidarisé avec le bandage par des parties de bandes plâtrées constituant ce dernier passant sous des parties de moindre épaisseur de l'élément d'appui disposées de part et d'autre de la partie en saillie comportant un corps en un matériau rigide dont au moins la surface supérieure correspond sensiblement à la forme et à la pointure du pied du patient et plusieurs surfaces d'appui constituées par des bandes transversales en saillie, les bandes plâtrées formant le bandage pouvant être engagées dans l'espace entre deux bandes transversales contigües, caractérisée en ce qu'elle présente un ergot arrière ayant une longueur sensiblement égale à la saillie des bandes transversales et qui est dirigé en oblique et vers l'extérieur à partir du bord latéral extérieur de la partie arrière du talon de la semelle.

Cet ergot de part et d'autre duquel passent les bandes plâtrées facilite de plus la pose du plâtre et le maintien en position des bandes jusqu'à la prise complète.

Pour améliorer le confort la surface supérieure de la semelle comporte un soutien de voûte plantaire et/ou une pelote métatarsienne.

La semelle conforme à l'invention comporte de préférence une semelle orthopédique et de propreté amovible constituant la surface supérieure de la semelle et formant le soutien de voûte plantaire et la pelote métatarsienne. Cette semelle de propreté amovible peut être intéressante du fait du caractère réutilisable de la semelle et de la nécessité de l'adapter à des pieds plus ou moins plats. Cette semelle de propreté peut être mince et réalisée en mousse de plastique.

La présente invention sera mieux comprise à la lecture de la description détaillée d'un mode de réalisation faite ci-après avec référence au dessin ci-annexé dans lequel:

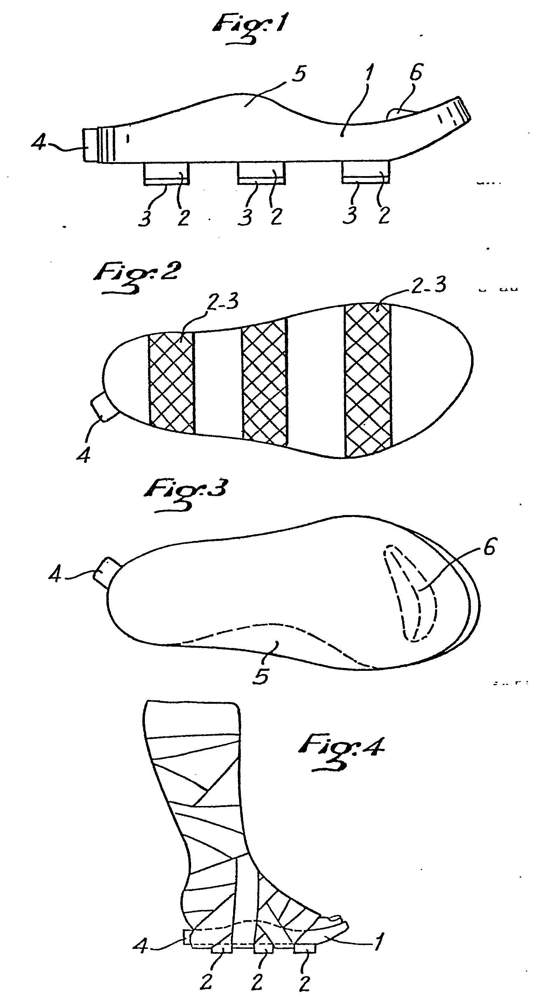

La figure 1 est une vue en élévation latérale de la semelle,
la figure 2 en est une vue par dessous,
la figure 3 une vue par dessus et
la figure 4 une vue en élévation latérale schématique d'une botte plâtrée utilisant la semelle.

La semelle conforme à l'invention comporte un corps 1 en polyuréthane rigide moulé avec, sous la surface inférieure de la semelle, trois bandes en saillie 2 dont la surface d'appui inférieure 3 est constituée par une bande antidérapante. Ces bandes ont une largeur approximative de 3 centimètres et leur écartement est du même ordre de grandeur, leur épaisseur étant de 15 à 20 millimètres.

A l'arrière du corps, à partir du bord arrière extérieur de la semelle et dirigé obliquement vers l'extérieur est prévu un ergot 4 qui fait saillie d'environ 20 millimètres par rapport au bord externe de la semelle.

Sur la surface supérieure de la semelle sont réalisés un soutien de voûte plantaire 5 et une pelote métatarsienne 6.

Comme illustré à la figure 4 le pied est posé directement sur la semelle et les bandes plâtrées sont enroulées de la manière usuelle en passant sous la semelle entre les bandes transversales 2 et à l'arrière entre le bord arrière de la bande transversale et les côtés de l'ergot 4.

## Revendications

1. Semelle pour la réalisation d'un bandage au plâtre du pied avec incorporation d'un élément d'appui pour la marche dont une partie fait saillie sous la surface inférieure du bandage, cet élément d'appui étant solidarisé avec le bandage par des parties de bandes plâtrées constituant ce dernier passant sous des parties de moindre épaisseur de l'élément d'appui disposées de part et d'autre de la partie en saillie comportant un corps (1) en un matériau rigide dont au moins la surface supérieure correspond sensiblement à la forme et à la pointure du pied du patient et plusieurs surfaces d'appui constituées par des bandes transversales en saillie (2), les bandes plâtrées formant le bandage pouvant être engagées dans l'espace entre deux bandes transversales contigües, caractérisée en ce qu'elle présente un ergot arrière (4) ayant une longueur sensiblement égale à la saillie des bandes transversales et qui est dirigé en oblique et vers l'extérieur à partir du bord latéral extérieur de la partie arrière du talon de la semelle.

2. Semelle selon la revendication 1, caractérisée en ce qu'elle comporte un soutien de voûte plantaire (5) et/ou une pelote métatarsienne (6).

3. Semelle selon l'une quelconque des revendication 1 et 2, caractérisée en ce qu'elle comporte une semelle orthopédique et de propreté amovible constituant la surface supérieure de la semelle et formant le soutien de voûte plantaire et la pelote métatarsienne.

## Claims

1. A sole for providing a cast bandage for the foot, comprising a walking bearing element, a portion of which is protruding underneath the lower surface of the bandage, said bearing element being rigidly connected to the bandage by portions of plastered band forming said bandage passing underneath portions of lesser thickness of the bearing element disposed on either side of the protruding portion comprising a body (1) in a rigid material, the upper surface of which at least corresponds substantially to the shape and size of the patient's foot and several bearing surfaces formed by protruding transverse bands (2), whereby the plastered bands forming the bandage can be engaged in the space between two contiguous transverse bands, characterized in that said sole is formed with a rear lug (4) having a length substantially equal to the projection of the transverse bands and which is extending obliquely and toward the outside from the outer lateral edge of the rear portion of the sole heel.

2. A sole according to claim 1, characterized in that it comprises a support (5) for the arch of the foot and/or metatarsal wad (6).

3. A sole according to any one of claims 1 and 2, characterized in that it comprises a removable orthopaedic and cleanliness sole constituting the upper surface of the sole and forming the support for the arch of the foot and the metatarsal wad.

## Patentansprüche

1. Sohle zur Herstellung eines Gipsverbandes für den Fuss mit Einbau eines Stützelementes für das Gehen, dessen ein Teilstück unter der unteren Oberfläche des Verbandes vorspringt, wobei dieses Stützelement mit dem Verband durch Teile von Gipsbinden verbunden ist, welche diesen letzteren bilden, wobei sie unter Partien von geringer Stärke des Stützelementes verlaufen, die beiderseits des vorspringenden Teilstücks angeordnet sind, das einen Körper (1) aus steifem Material, zumindest dessen Oberseite annähernd der Form und der Grösse des Fusses des Patienten entspricht, und mehrere Auflageflächen aufweist, die durch vorspringende Querstreifen (2) gebildet sind, wobei die Gispbinden, die den Verband bilden, in den Zwischenraum zwischen zwei benachbarten Querstreifen eingesetzt sein können, dadurch gekennzeichnet, dass sie einen hinteren Vorsprung (4) aufweist, der eine Länge annähernd gleich dem Vorsprung der Querstreifen hat und der schräg nach aussen ausgehend vom seitlichen äusseren Rand des hinteren Teilstücks des Absatzes der Sohle gerichtet ist.

2. Sohle nach Anspruch 1, dadurch gekennzeichnet, dass sie eine Stütze für die Fusswölbung (5) und/oder ein Mittelfusskissen (6) aufweist.

3. Sohle nach einem der Ansprüche 1 oder 2, dadurch gekennzeichnet, dass sie eine orthopädische und wegen der Sauberkeit auswechselbare Einlage aufweist, die die obere Oberfläche der Sohle bildet und die die Stütze für das Fussgewölbe und das Mittelfusskissen bildet.

0 064 451

*Fig.1*

*Fig.2*

*Fig.3*

*Fig.4*

5